# EUROPEAN PATENT APPLICATION

(11) **EP 3 723 097 A1**
(43) Date of publication of application: **14.10.2020**
(21) Application number: 20168484.2
(22) Date of filing: 07.04.2020
(51) Int. Cl.: G16H 20/13

(54) **METHOD FOR PERSONALISED MANAGEMENT OF PHARMACOLOGICAL THERAPY**

(30) Priority: 10.04.2019 IT 201900005496
(71) Applicant: Demax Depositi e Trasporti SpA, 00071 Pomezia (RM) (IT)
(72) Inventor: STASI, GEMMA, I-00071 POMEZIA (RM) (IT)
(74) Representative: Fiammenghi, Eva

(57) **Abstract**

Method for personalised management of pharmacological therapy comprising the step (100) for identifying the patient with the detection of biometric data and the release of a unique identification code, the step (200) for computerised prescription of the therapy by the prescriber, the step (300) for preparing the medicinal product in a local facility authorised for the purpose, the step (400) for distributing the medicinal product directly to the home of the patient or in the neighbourhood computerised vending machine cabinet (410), the step (500) for purchasing the medicinal product by taking the medicinal products from said vending machine cabinets and the step (600) for taking the medicinal product wherein the patient sends the specific bar code (354) relating to the dose taken by a common client terminal (610) as well as a smartphone or an optical reader suitable for the purpose to the server terminal (110) of the prescriber.

## Description

### Field of the invention

The present invention patent application regards the health industry and more particularly the field of pharmacology and pharmacotherapy.

The invention regards an innovative method for the prescription, logistics and dispensing of drugs.

### State of the Art

As is well known, ageing of the population is a globally emerging phenomenon, both in developing countries and in developed countries.

This phenomenon is particularly evident in Italy, where it is expected that in 2050, subjects over 65 years of age will represent 34.4% of the total population and subjects over 80 years of age will represent 14.2% of the total population. These figures are considerable if we consider that in 2001 those over sixty-five years of age were 18.2% and those over eighty years of age represented 4.1% of the total population.

But, while longevity is in itself a breakthrough, on the one hand ageing populations have many economic, social, and welfare implications. The increasing number of elderly people is increasing the demand for social and health services and treatment. The consequences of such an intense and rapid ageing are numerous and with reference to the national health system, these are attributable to a dramatic increase in health and care costs, both due the increased number of hospital admissions and, above all, due to the increased need for long-term care as well as due to the increase in pharmaceutical spending.

Therefore, the significant demographic and epidemiological changes that characterise this phase of the socio-health journey in our country have now led to the need for a significant redefinition of the type of response to a question that largely revolves around the health and social health needs of the population.

Although considerable efforts have been made with the advent of information technology to reduce and rationalise health spending, for example the introduction of the electronic prescription, health deficits countering measures, and attempt to control spending on goods and services, the introduction of regional health-care companies, and so on, promising results toward markedly cutting health spending have not yet been achieved up to date, solely leading to an inevitable drop in the quality of the services provided.

The present invention was precisely conceived with the aim of addressing the need for reducing health-care costs and more specifically in the management of medicinal products and not alongside an inexorable reduction of the services provided but rather alongside innovative aspects that translate into a better attention to the needs of patients. This assertion stems from the fact that the present invention tends to direct the patient toward a careful individual management of the pharmacological therapy, in particular facilitating all actions ranging from purchasing the medicinal product to its correct intake, which, as implied above, represent the critical stages in the case of a predominantly elderly population.

Other advantages of the invention will be apparent from the detailed description of an embodiment thereof, provided by way of non-limiting example, illustrated below.

### Description of the invention

The present industrial invention patent application aims at describing and claiming an innovative method for personalised management of pharmacological therapy.

More specifically, the present invention provides for creating an innovative logistics system using a common computerised medicinal product vending machine cabinet, which is highly efficient and at the same time considerably cost-effective and therefore capable of eliminating or at least reducing the aforementioned drawbacks.

Another innovative concept on which the present invention is based consists in the provision of a particular container for medicinal products as well as a particular process suitable to allow both the patient to comply with the medical prescription and to monitor and control whether or not the patient follows the medical prescription.

As known, the treatment of many diseases provides for several medicinal products which, in order to guarantee the success of the treatment, must be taken according to a given sequence/frequency and, in particular, at predetermined times of the day. For these reasons, the patient must store a large number of dose-related information and therefore risks to easily forget hence leading to reducing the effectiveness of the treatment and, in some cases, rendering the treatment almost ineffective, with negative health-related consequences, even lethal in the case of life-saving medicinal products. Such observance of the medical prescription by the patient, more technically referred to as compliance, is optimised in the present invention thanks to two characteristics. On the one hand, the computer technology used, provided in the form of common hardware and software, as well as hardware and software components already widely available in the management of the medicinal product in the nosocomial field, allows the physician to verify in real time at any time whether or not the patient is complying with the treatment prescribed thereto. On the other hand, the use in the method of an inventive packaging method for medicinal products allows the patient to be guided correctly and assisted in the correct intake of pharmacological therapy.

Therefore, to the full advantage of practicality and cost-effectiveness, the fundamental objects included in the method for personalised management of pharmacological therapy according to the present invention are tripled and consist in creating a model of optimisation of the logistics of the medicinal product and in particular creating the step for the smart dispensing thereof, in allowing to monitor the patient's compliance with medical prescriptions and lastly in facilitating the patient in the management of medical therapy and in particular by assisting the patient to take the medicinal product.

Therefore, from the above, the inventive concept certainly represents a very useful tool toward addressing a new challenge capable of improving the services of the health care facilities to the citizen as well as reducing the risks and costs related with the patient's care. Other characteristics of the present invention are described in the following detailed description of one or more specific embodiments, protected by the various dependent claims also with reference to the Figures attached and outlined below.

### Brief description of the figures

Further characteristics and advantages of the present invention will become more apparent from the following detailed description, provided by way of non-limiting example and illustrated in the attached Figures, wherein:
- FIGURE 1 illustrates the block diagram of the method of the invention;
- FIGURE 2 illustrates a top perspective view of the modular container of the invention with the first shelf raised and partially removed;
- FIGURE 3 illustrates a top perspective view of the modular container of the invention with the hidden corners drawn with dashed lines and the front surface removed;
- FIGURE 4 illustrates the data sharing diagram in the client/server system according to the present invention.

### Detailed description of the invention

The present description and the Figures attached herein are to be considered as illustrative and non-limiting with respect to the present invention, which can be implemented according to other and different embodiments, still falling within the scope of protection of the attached claims, which also form an integral part of the text.

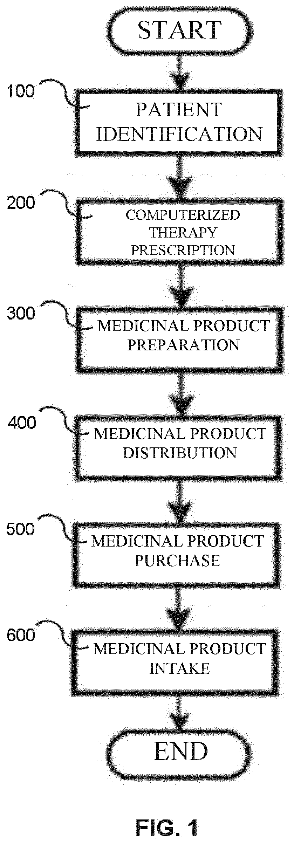

With reference to Fig. 1, said method of the invention of personalised management of the pharmacological therapy generally consists of the following constituting steps, which, as can be seen, reduce all equipment and people that still fall within the management of pharmacological therapy up to date:
1. patient identification step 100;
2. computerised therapy prescription step 200;
3. medicinal product preparation step 300;
4. medicinal product distribution step 400;
5. medicinal product purchase step 500;
6. medicinal product intake step 600.

In brief, the aforementioned steps represent the entire method of the invention, which however in the following description will be expounded and analysed in detail.

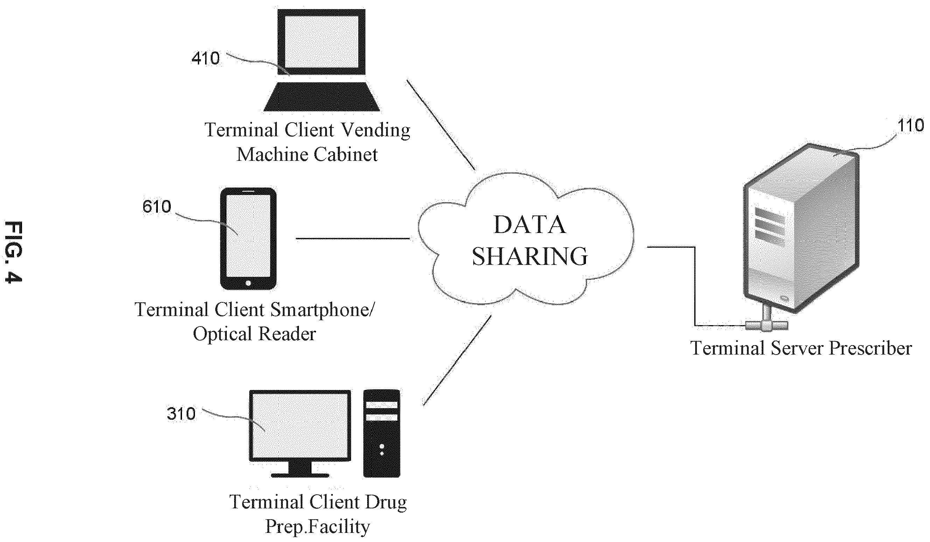

The patient identification step 100 begins when the patient goes to a medical facility where the server terminal 110 is preferably kept which, as shown in Fig. 4, represents the core of the computer management of the entire method of the invention by creating a common client/server computer system. This is where the first actual patient identification procedure on a terminal or on the server terminal 110 begins, by detecting at least one biometric data as well as the fingerprint, colour and size of the iris, the physiognomy of the face and so on, in order to allow biometric recognition. Once the data has been acquired, the patient is assigned a unique identification code in the form of a bar code or QR code and the relative ciphering. Then, next follows the computerised therapy prescription step 200, wherein after conducting the anamnesis, after identifying the symptoms and after visiting the patient, the physician defines a diagnostic hypothesis and prescribes a pharmacological therapy deemed most appropriate through the computerised form contained in the terminal thereof or on the server terminal 110, indicating the type of drug, duration, intake times, dose thereof and obviously associating it with the unique identification code of the patient.

At this point next is the medicinal product preparation step 300 which is carried out by sharing the prescribed pharmacological therapy with the client terminal 310 of the local facility designated for the preparation and definition of the pharmacological therapy.

At this point it should be pointed out that in all the embodiments of the invention, the general fundamental principle for the purposes of monitoring patient compliance consists in the fact based on the invention that one or more doses of medicinal products which can be taken simultaneously at a given time of day must be placed in at least one container capable of housing them and keeping, therein, a unique barcode or QR code 354 for that specific administration and so that such code cannot be viewed unless after opening the container.

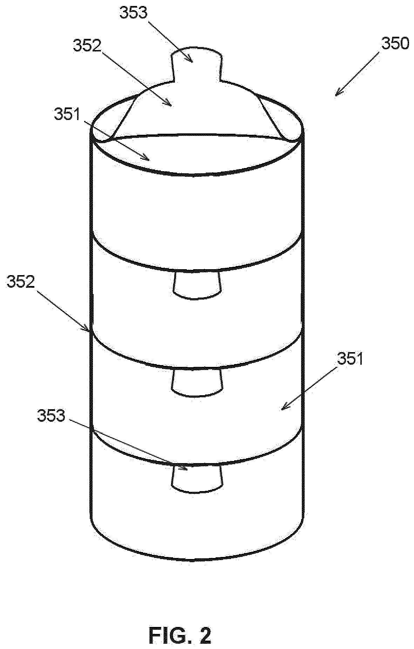

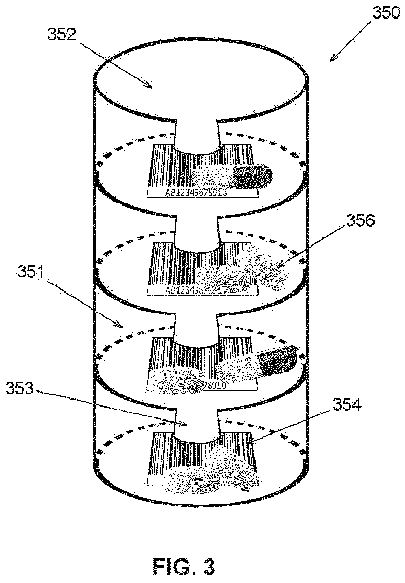

In the light of the above, it is provided for that in the case of prescriptions of therapies consisting of one or more doses of medicinal products which can be taken simultaneously, which as a whole occupy a volume comprised between 3 cm³ and 17 cm³ and preferably between 6 cm³ and 14 cm³, an innovative modular container 350 according to Figures 2 and 3, which for convenience of packaging and monitoring of intake preferably contains all doses to be taken within 24 hours, is used. In particular, a common packaging machine arranged in the local facility designated for the preparation and definition of the pharmacological therapy provides for the packaging of said innovative modular container 350 containing the aforementioned doses of the medicinal product 360.

As shown in Figs. 2 and 3, said modular container 350 is preferably cylindrical-shaped and consists of several compartments 351 arranged one over the other and separated from each other by removable shelves 352, by gripping and pulling the corresponding segmented tabs 353 upwards, facilitating the opening. Each of said compartments 351, arranged to contain one or more doses of medicinal product 356 which can be taken simultaneously at a given time of the day, will be shaped so as to allow the use of the doses positioned in the compartment 351 located at the topmost. Therefore, as observable in Fig. 3, once the compartment 351 arranged at the topmost position is opened, the shelf 352 which can be highlighted, that is to say the one on which one or more doses of the medicinal product 356 to be taken at that moment, are placed, will detect the relative barcode or QR code 354 identifying the patient code, trade name or active ingredient of the drug, the expiration date, the packaging date, the time of administration.

The medicinal product distribution step 400 consists in the fact that after defining the pharmacotherapy, the availability of all the aforementioned medicinal products to the patient will be carried out according to any of the following two methods, which consist in the direct delivery to the patient's home or in the storage by the designated staff in appropriate cabinets for the neighbourhood computerised vending machine cabinets 410 and thus placed in predefined zones of each area of the cities.

The next medicinal product purchasing step 500 or the step in which the above medicinal products are made available for the patient, may thus occur either directly in the case of delivery at home or by the patient going to the neighbourhood computerised vending machine cabinet 410 and authenticating him/herself by means of the biometric system or by entering the unique identification code or alternatively by nearing the barcode or QR code thereof. It is understood that the amount of doses of medicament delivered by said neighbourhood computerised vending machine cabinet 410 over a given period will comply with what is set by the prescriber in the aforementioned step 200.

The medicinal product intake step 600 represents the most sensitive step of the method of the invention since it comprises the step for monitoring the patient's intake by the prescriber. Once the medicinal products are available, at the time of intake the patient will open the container or compartment 351 of the modular container 350 and will take the medicinal product or medicinal products contained therein. Such operation will detect the barcode or QR code 354 contained in the compartment 351, which the patient will promptly read through a client terminal 610 and then, based on what is shown in Fig. 4, said data will automatically be shared with the server terminal 110 of the physician. In this manner, the physician will have the latest update on the patient's condition as concerns whether the medicinal product has been taken and whether this has been done correctly.

Even though the above description generally represents the method of the invention, for the sake of the completeness and versatility of the present invention, further preferred embodiments at the medicinal product intake step 600 are provided for. As a matter of fact, in such step several solutions which are capable of making said method of the invention applicable to several conditions that can be attributed to almost all the treated patients are provided for.

Although a common client terminal 610 has been described so far as in Fig. 4, it should be observed that there are several variants at this level, given that they will inevitably depend on the level of learning and computerisation of the patient as well as the availability of resources in the patient's environment. Firstly, the simplest and most cost-effective means coinciding with the aforementioned client terminal 610 is a common smartphone which already represents a hardware platform on which it is easy to run by simply reading the barcode or QR code 354 associated with a particular dose by means of a simple application and then sharing the collected data with the server terminal 110 of the physician.

In cases where it is not possible to use a smartphone, during the aforementioned medicinal product purchase step 500 the patient can also be provided with an electronic device, in the form of an optical bracelet reader, capable of performing the client terminal 610 function and reading the barcode or QR code 354. In this case, the step of sharing the collected data with the server terminal 110 of the physician requires that the patient goes to the neighbourhood computerised vending machine cabinet 410 and connects his/her optical bracelet reader to the appropriate space, in order to download the intake data for the stored drugs.

Finally, the patient, notwithstanding the description outlined above, can connect to a dedicated telephone number by means of a common telephone and enter the aforementioned identification codes indicated below the bar code or QR code 354 of each single dose. Though the description outlined above generally represents the method of the invention, the present invention provides for further alternative embodiments regarding some variants of its constituent steps and of the type of components thereof.

In particular, the client terminal 610, whether it be a smartphone or an optical reader, can perform the useful function of alerting when the time to take the medicinal product has reached, based on the dose set by the physician, by emitting acoustic and/or light signals.

A further alternative embodiment, reported for the sake of completeness of the entire method of the invention, provides for that the neighbourhood vending machine cabinets, in the case of emergency or in the impossibility for the patient to reach the prescriber, may deliver a maximum number of doses of the therapy so as to meet the intake requirements up to a maximum of 60 hours, beyond which the dispensing period will be blocked if not carried out by means of the aforementioned computerised therapy prescription step 200. Logically, all these requirements notwithstanding the system of normal computerised prescription and in any case any anomalies not remedied in the prescribed times and ways will be reported in the prescriber's directory.

Though the present invention has been described by way of non-limiting example, according to some preferred embodiments, it should be observed that any variations and/or modifications may be carried out by a man skilled in the art without departing from the relative scope of protection, as defined by the following dependent claims.

## Claims

1. Method for personalised management of pharmacological therapy **characterised in that** it comprises:
a. the step (100) for identifying the patient wherein the latter goes to a medical facility where the server terminal (110) is present, part of a common client/server computer system, through which the identification thereof is sent by detecting at least one biometric data same case applying to the fingerprint, the colour and the size of the iris, the physiognomy of the face and wherein a unique identification code in form of bar code or QR code and the relative ciphering are allocated to the patient;
b. the step (200) for computerised therapy prescription wherein after carrying out the anamnesis, identifying the symptoms and examining the patient, the physician draws a diagnosis and prescribes the pharmacological therapy deemed most appropriate, through the computer module in use on said server terminal (110), indicating the type of drug, duration, intake times, dose, maximum amount of medicinal product required for a determined period and obviously associating it to the unique identification code of the patient;
c. the step (300) for setting up the medicinal product wherein the pharmacological therapy prescribed in the previous step is shared with the client terminal (310) of the local facility designated for preparing and setting up the pharmacological therapy and wherein the one or more doses of medicinal product (356) part of the therapy and that can be taken simultaneously, are kept in a single shared container, which reveals the relative barcode or QR code (354) identifying the code of the patient, the trade name or the active ingredient of the drug, the date of expiration, the date of packaging and the scheduled administration time only after opening.
d. the step (400) for distributing the medicinal product wherein the direct delivery of the shared containers containing the doses of medicinal product to the home of the patient or storage thereof by the designated personnel in special neighbourhood computerised vending machines (410), i.e. kept in predefined areas in each single area of the town, occurs;
e. the step (500) for purchasing the medicinal product wherein the aforementioned medicinal products are directly made available for the patient, in case of home delivery, or by the patient by going to the neighbourhood computerised vending machine cabinet (410) and authenticating him/herself through the biometric system or by entering the unique identification code or alternatively by nearing the barcode or QR code thereof; and
f. the step (600) for taking the medicinal product which occurs when, at the time of intake by the patient, the patient opens the aforementioned shared container and takes one or more doses of medicinal product (356) contained therein, thus revealing the unique barcode or QR code (354) for that specific intake, which will be promptly read by said patient through a client terminal (610) and then shared with the server terminal (110) of the prescriber through a common data sharing protocol, thus making the aforementioned physician aware of the updated situation of the patient as regards the occurred and correct intake of the medicinal product.

2. Method according to any one of the preceding claims, **characterised in that** in the step (600) for the intake of the medicinal product the client terminal (610) used is a common smartphone capable, by means of a simple application, of reading the barcode or the QR code (354) associated to a particular dose of the medicinal product (356) to be taken and thus share the collected data through the server terminal (110) of the physician.

3. Method according to any one of the preceding claims, **characterised in that** in the step (600) for taking the medicinal product the function of the client terminal (610) is carried out by an electronic device in form of an optical bracelet reader, capable of reading the barcode or the QR code (354) associated to a particular dose of medicinal product (356) and **in that** the patient shares the collected data with the server terminal (110) of the physician by going to the neighbourhood computerised vending machine cabinet (410) and that the latter connects said optical bracelet reader in the special space, so as to download the stored drugs intake data.

4. Method according to any one of the preceding claims, **characterised in that** in the step (600) for taking the medicinal product the patient may be directly connected, by means of a common telephone, to a dedicated telephone number and enter the aforementioned identification codes indicated below the barcode or QR code (354) of each single taken dose of medicinal product (356).

5. Method according to any one of the preceding claims, **characterised in that** in the step (600) for taking the medicinal product the client terminal (610) used, whether a smartphone or an optical reader, emits a sound or light warning signal when the patient is scheduled to take the medicinal product and **in that** the warning is emitted based on the dosage set by the prescriber.

6. Method according to any one of the preceding claims, **characterised in that** in case of emergency, or should the patient not be in a position to go to the prescriber, the neighbourhood computerised vending machine cabinet (410) can dispense a number of doses of medicinal product (356) of the therapy, so as to provide for the intakes up to a maximum of 60 hours and **in that** all said prescriptions exempted from the common computerised therapy prescription (200) will be signalled in the database of the prescriber.

7. Method according to any one of the preceding claims, **characterised in that** in case of prescription of therapies consisting of one or more doses of medicinal products that can be taken simultaneously, that as a whole occupy a volume comprises between 3 cm³ and 17 cm³ and preferably between 6 cm³ and 14 cm³, when setting up the medicinal product (300) the latter are kept in a single compartment (351) of a modular container (350) and **in that** each modular container (350) contains all compartments (351) containing the single doses of medicinal product (356) that can be taken in the span of 24 hours.

8. Method according to any one of the preceding claims, **characterised in that** said modular container (350) consisting of compartments (351) containing the doses of medicinal product (360) is packaged in the step of setting up the medicinal product (300) by means of a common packaging machine arranged in the aforementioned local facility designated for preparing and setting up the pharmacological therapy.

9. Container according to any one of the preceding claims, **characterised in that** it comprises a cylindrical-shaped modular container (350), consisting of several compartments (351) arranged one on top of the other and mutually separated by shelves (352) that can be removed by gripping and pulling upwards the relative segmented tabs (353) facilitating the opening, **in that** each of said compartments (351), designed to contain one or more doses of medicinal product (356) that can be take simultaneously at a given time of the day, will be configured so as to allow the use of the doses positioned in the compartment (351) positioned topmost, **in that** once opened said compartment (351) positioned topmost highlights the shelf (352), i.e. the one on which the one or more doses of medicinal product (356) to be taken at that time lies, which detects the relative barcode or QR code (354) identifying the code of the patient, the trade name or active ingredient, the date of expiration, the date of packaging and the scheduled administration time.
